# EUROPÄISCHE PATENTSCHRIFT

(11) **EP 0 888 384 B1**
(45) Veröffentlichungstag und Bekanntmachung des Hinweises auf die Patenterteilung: **11.12.2002**
(21) Anmeldenummer: 97919258.0
(22) Anmeldetag: 07.03.1997
(51) Int. Cl.: C07K 14/00

(54) **FUSIONSPOLYPEPTID ZUR BEEINFLUSSUNG VON WECHSELWIRKUNGEN ZWISCHEN PROTEINEN**
FUSION POLYPEPTIDE FOR MODIFYING INTERACTIONS BETWEEN PROTEINS
POLYPEPTIDE DE FUSION POUR INFLUER SUR LES INTERACTIONS ENTRE DES PROTEINE

(30) Priorität: 07.03.1996 DE 19608813
(43) Veröffentlichungstag der Anmeldung: 07.01.1999
(73) Patentinhaber: Röwekamp, Walter, 69123 Heidelberg (DE); Rose-John, Stefan, 55129 Mainz (DE)
(72) Erfinder: ROSE-JOHN, Stefan, J. Gutenberg-Universität Mainz, 55101 Mainz (DE)
(74) Vertreter: Schüssler, Andrea, Dr.
(86) Internationale Anmeldenummer: DE9700458
(87) Internationale Veröffentlichungsnummer: WO97032891

(56) Entgegenhaltungen:
- WO-A-95/15341
- WO-A-96/04314
- US-A- 5 260 203
- STOYAN T. ET AL.: "Recombinant soluble human interleukin-6 receptor" EUROPEAN JOURNAL OF BIOCHEMISTRY, Bd. 216, Nr. 1, 11.August 1993, Seiten 239-245, XP002047601
- EHLERS M ET AL: "IDENTIFICATION OF TWO NOVEL REGIONS OF HUMAN IL-6 RESPONSIBLE FOR RECEPTOR BINDING AND SIGNAL TRANSDUCTION" JOURNAL OF IMMUNOLOGY, Bd. 153, Nr. 4, 15.August 1994, Seiten 1744-1753, XP000565715
- SUI, X. ET AL.: "gp130 and c-Kit signalings synergize for ex vivo expansion of human primitive hemopoietic progenitor cells" PROCEEDINGS OF THE NATIONAL ACADEMY OF SCIENCES OF USA., Bd. 92, März 1995, WASHINGTON US, Seiten 2859-2863, XP002047602
- FISCHER, M. ET AL.: "A bioactive designer cytokine for human hematopoietic progenitor cell expansion" NATURE BIOTECHNOLOGY., Bd. 15, Nr. 2, Februar 1997, UBLISHING US, Seiten 142-145, XP002047603

## Beschreibung

Die vorliegende Erfindung betrifft ein Fusionspolypeptid, daß sich zur Beeinflussung von Wechselwirkungen zwischen Proteinen eignet, eine ein solches Fusionspolypeptid kodierende DNA und die Verwendung des Fusionspolypeptids.

Viele Vorgänge in einem Organismus beruhen auf Wechselwirkungen zwischen Proteinen. Beispiele solcher Wechselwirkungen finden sich bei Rezeptoren und den an sie bindenden Liganden. Oftmals sind allerdings die Wechselwirkungen zwischen Proteinen gestört. Dies kann daran liegen, daß einzelne an den Wechselwirkungen beteiligte Proteine modifiziert sind, wodurch ihre Affinität zu anderen, ebenfalls beteiligten Proteinen verändert ist. Auch können einzelne an den Wechselwirkungen beteiligte Proteine fehlen. Dies findet man z.B. bei Zellen, die nicht auf Interleukin-6 (IL-6) reagieren. Solche Zellen weisen einen unvollständigen Interleukin-6-Rezeptor auf, d. h. dieser Rezeptor umfaßt lediglich die intrazelluläre, Signal-auslösende Untereinheit gp130, nicht aber die extrazelluläre, IL-6 bindende Untereinheit (IL-6R).

Viele Versuche werden unternommen, gestörte Wechselwirkungen zwischen Proteinen zu beheben. Beispielsweise wird dies bei einem unvollständigen Interleukin-6-Rezeptor durch Verabreichung von IL-6 (50 ng/ml) und löslichem IL-6R (sIL-6R) (1280 ng/ml) versucht. Die Bereitstellung von sIL-6R bedingt jedoch einen großen Kosten- und Zeitaufwand, da slL-6R nur biologisch aktiv ist, wenn es aus eukaryotischen Zellen stammt, und die Erträge aus solchen im Bereich von 1-6 mg sIL-6R/I liegen. Die genannte Verabreichung stellt somit kein geeignetes Mittel dar, dauerhaft die gestörten Wechselwirkungen bei einem unvollständigen Interleukin-6-Rezeptor zu beheben.

Aus der WO-A 96/04314 ist ein MHC-Molekül bekannt, an das ein zu präsentierendes Pepid kovalent gebunden ist. Ferner gehen aus European Journal of Biochemistry, Band 216, Nr. 1 (1993) 239-245 und Journal of Immunology, Band 153, Nr. 4 (1994), 1744-1753 jeweils ein cross-linking von IL-6 und IL-6-Rezeptor hervor. Des weiteren weist Proc. Natl. Acad. Sci., U.S.A., Band 92 (1995), 2859-2863 darauf hin, daß die Zugabe von IL-6 und IL-6-Rezeptor sowie Stammzellfaktor zu einer Stimulation der Expansion von haematopoetischen Vorläuferzellen bzw. von CD34⁺-Zellen führt.

Der vorliegenden Erfindung liegt die Aufgabe zugrunde, ein Mittel bereitzustellen, mit dem gestörte Wechselwirkungen zwischen Proteinen, insbesondere bei einem unvollständigen Interleukin-6-Rezeptor, behoben werden können.

Erfindungsgemäß wird dies durch die Gegenstände in den Patentansprüchen erreicht.

Gegenstand der vorliegenden Erfindung ist somit ein Fusionpolypeptid, das zwei, eine Affinität zueinander aufweisende, Polypeptide umfaßt, wobei das eine Polypeptid ein Zytokin der Interleukin-6-Familie und das andere der dazugehörige Rezeptor ist und die Polypeptide über einen Polypeptid- Linker miteinander verbunden sind.

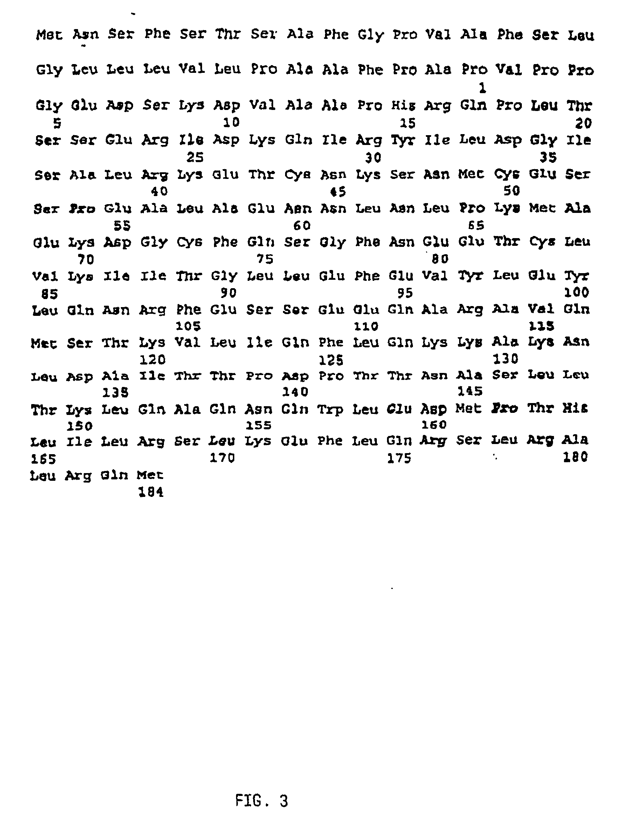

Der Rezeptor kann in Form seiner Untereinheit bzw. des funktionellen Teils davon vorliegen, die den Liganden binden. Ebenso kann der Ligand in Form seiner Untereinheit bzw. des funktionellen Teils davon vorliegen, die den Rezeptor binden. Vorzugsweise ist der Rezeptor ein Interleukin-6-Rezeptor oder ein CNTF-Rezeptor. Entsprechendes gilt für den Liganden. Dieser ist ein Mitglied der Interleukin-6-Familie, insbesondere IL-6, IL-11, CNTF, OSM, LIF oder CT-1. Der Rezeptor und der Ligand können Wildtyp-Sequenzen oder hiervon durch ein oder mehrere Nukleotide unterschiedliche Sequenzen umfassen. Dadurch können der Rezeptor und der Ligand verbesserte und/oder neue Eigenschaften aufweisen. Verbesserte Eigenschaften können z.B. darin liegen, daß die Bindung zwischen dem Rezeptor und dem Ligand verbessert ist. Neue Eigenschaften können z.B. darin liegen, daß der Ligand ein verändertes Verhalten zu Proteinen zeigt, mit denen er nach Bindung an den Rezeptor reagiert. Beispielsweise kann IL-6 dahingehend verändert sein, daß es eine stärkere Bindung an den IL-6-Rezeptor hat, das Protein gp130 aber nicht mehr aktivieren kann. In einem solchen Fall umfaßt IL-6 vorzugsweise die Sequenz von Fig. 3 oder Fragmente davon. Vorstehende Ausführungen hinsichtlich einer Veränderung der Wildtyp-Sequenz eines Rezeptors bzw. eines Liganden gelten entsprechend für deren Untereinheiten und funktionellen Teile davon, die zu einer gegenseitigen Bindung beitragen.

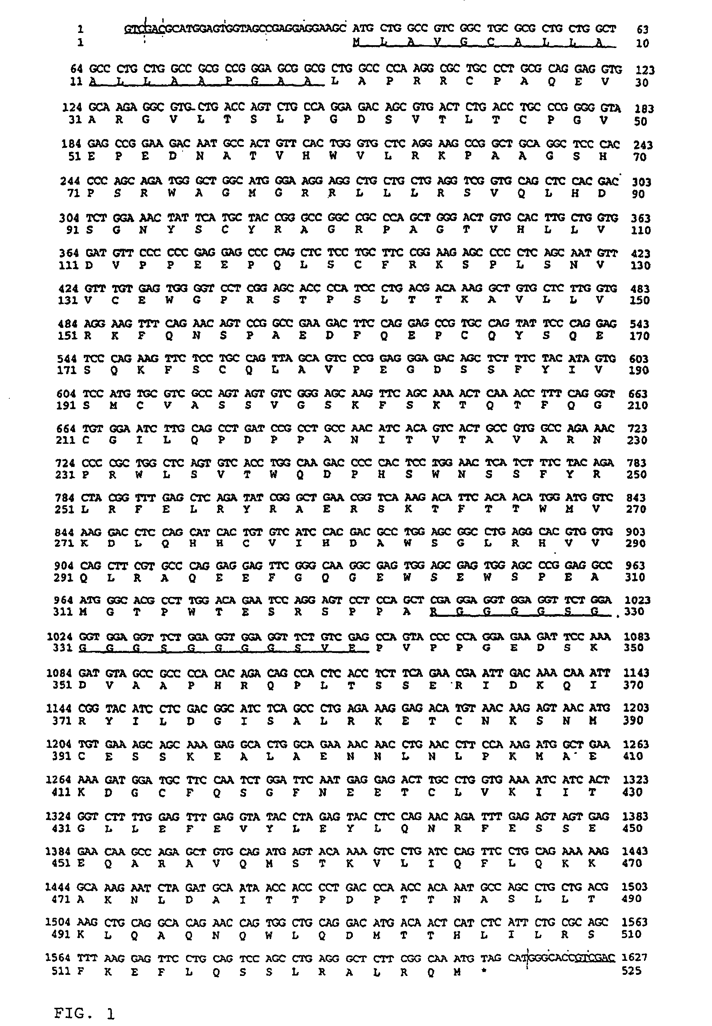

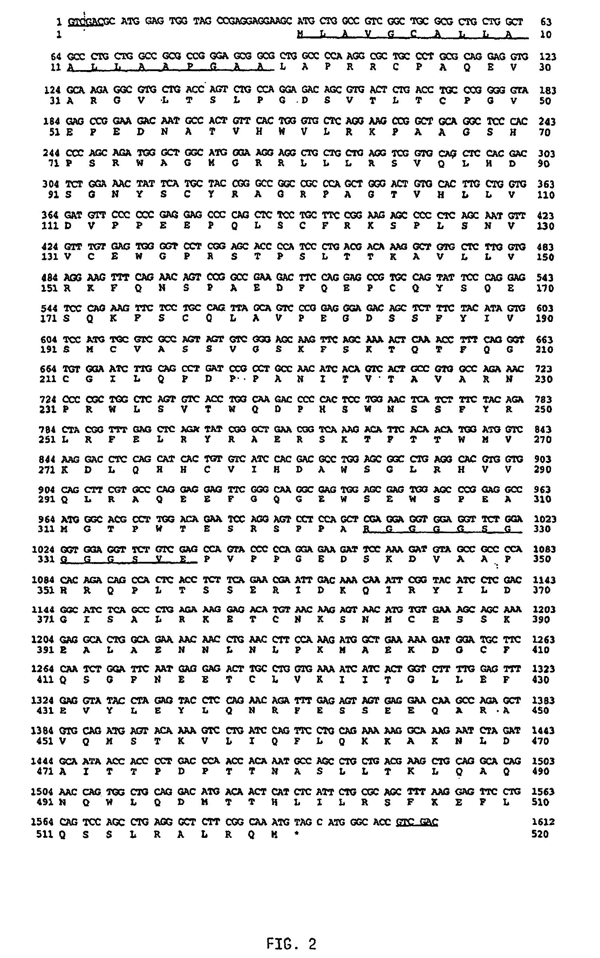

Beispiele eines erfindungsgemäßen Fusionspolypeptids sind in den Figuren 1 und 2 angegeben. Diese Fusionspolypeptide umfaßen ein humanes sIL-6R-Polypeptid, d.h. die extrazelluläre Untereinheit eines Interleukin-6-Rezeptors, und ein humanes IL-6-Polypeptid, wobei die Polypeptide über unterschiedliche Polypeptid-Linker miteinander verbunden sind. Diese Fusionspolypeptide werden mit H-IL-6 bezeichnet. Eine Variation von H-IL-6, die von dem sIL-6R-Polypeptid nur die Aminosäuren Pro 114 bis Ala 323 enthält, wird ebenfalls bereitgestellt. Ferner wird eine Variation von H-IL-6 bereitgestellt, weiche die Aminosäuren 113 bis 323 des sIL-6R-Polypeptids und die Aminosäuren 29 bis 212 des IL-6-Polypeptids umfaßt. Desweiteren wird ein Fusionspolypeptid H-IL-6 bereitgestellt, dessen IL-6-Polypeptid die Sequenz von Fig. 3 umfaßt. Das sIL-6R-Polypeptid dieses Fusionspolypeptids umfaßt eine vollständige Sequenz bzw. die Sequenz zwischen den Aminosäuren 113 (114) bis 323 eines slL-6R-Polypeptids. Darüberhinaus wird ein Fusionspolypeptid bereitgestellt, das die extrazelluläre Untereinheit eines humanen CNTF-Rezeptors und humanes CNTF umfaßt, wobei beide Polypeptide über einen Polypeptid-Linker miteinander verbunden sind.

Ein weiterer Gegenstand der vorliegenden Erfindung ist eine für ein vorstehendes Fusionspolypeptid kodierende DNA. Ein Beispiel dieser DNA ist in Fig. 1 angegeben. Diese DNA wurde bei der DSM (Deutsche Sammlung von Mikroorganismen und Zellkulturen) als CDM8-H-IL-6 unter DSM 10549 am 27. 2. 1996 hinterlegt.

Eine erfindungsgemäße DNA kann in einem Vektor bzw. Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100, Ycpad1 und Vektoren für Pichia pastoris zu nennen, wobei letztere bevorzugt sind, während für die Expression in tierischen Zellen, die in einem Organismus oder außerhalb eines solchen vorliegen können, z.B. pKCR, pEFBOS, pCEV4 und pCDM8 anzugeben sind, wobei letzterer bevorzugt ist. Für die Expression in Insektenzellen eignet sich besonders der Baculovirus-Expressionsvektor pAcSGHisNT-A. Der Fachmann wird berücksichtigen, daß für die Expression einer erfindungsgemäßen, slL-6R-Sequenzen enthaltenden, DNA Vektoren angeraten sind, die eine Expression in eukaryotischen Zellen ermöglichen.

Ferner kennt der Fachmann geeignete Zellen, um eine erfindungsgemäße, in einem Expressionsvektor vorliegende DNA zu exprimieren. Beispiele solcher Zellen umfassen die E.coli-Stämme HB101, DH1, x1776, JM101, JM 109, BL21 und SG 13009, den Hefe-Stamm Saccharomyces cerevisiae und Pichia pastoris, wobei letzterer bevorzugt ist, die tierischen Zellen L, 3T3, FM3A, CHO, Vero, HeLa und COS, wobei letztere bevorzugt sind, sowie die Insektenzellen sf9.

Desweiteren weiß der Fachmann, in welcher Weise eine erfindungsgemäße DNA in einen Expressionsvektor inseriert werden muß. Auch kennt er Bedingungen, Zellen zu transformieren bzw. transfizieren und diese dann zu kultivieren. Darüberhinaus sind ihm Verfahren bekannt, das durch die erfindungsgemäße DNA exprimierte Fusionspolypeptid zu isolieren und zu reinigen.

Mit der vorliegenden Erfindung ist es möglich, die Wechselwirkungen zwischen Proteinen zu beeinflussen. Dies kann durch die Verabreichung erfindungsgemäßer Konjugate wie auch durch den Einsatz erfindungsgemäßer DNA in einer Gentherapie erfolgen. Insbesondere können die gestörten Wechselwirkungen bei einem unvollständigen Interleukin-6-Rezeptor behoben werden. Die vorliegende Erfindung zeichnet sich dadurch aus, daß sie kostengünstig eingesetzt werden kann. Dies zeigt sich insbesondere in der Verabreichung erfindungsgemäßer Konjugate zur Beeinflussung der gestörten Wechselwirkungen bei einem unvollständigen Interleukin-6-Rezeptor.

Ferner eignet sich die vorliegende Erfindung zur ex vivo Expansion von Stammzellen, insbesondere humanen Stammzellen. Besonders bemerkenswert ist es dabei, daß mit einem erfindungsgemäßen Konjugat H-IL-6 mehr Stammzell-Kolonien im Soft-Agar erhalten werden, als dies mit den einzelnen Komponenten IL-6 und sIL-6R möglich ist. Die vorliegende Erfindung stellt somit auch einen wichtigen Beitrag dar, gezielt in die Bildung von Blutzellen einzugreifen.

Desweiteren stellt die vorliegende Erfindung mit einem Fusionspolypeptid H-IL-6, das als IL-6-Polypeptid die Sequenz von Fig. 3 umfaßt, ein Mittel bereit, das sich als IL-6-Rezeptor-Antagonist eignet. Ein solches Mittel ist von hohem therapeutischen Wert.

Die Durchführung der vorliegenden Erfindung kann durch die erfindungsgemäßen Antikörper überwacht werden.

### Kurze Beschreibung der Zeichnung

- Fig. 1: zeigt die Aminosäure (DNA)-sequenz eines erfindungsgemäßen Fusionspolypeptids H-IL-6. Sequenzen für das Restriktionsenzym Sall (GTCGAC), das Signalpeptid (MLAVGCALLAALLAAPGAA) und den Linker (RGGGGSGGGGSGGGGSVE) sind angegeben. Der Linker verbindet den COOH-Terminus von humanem slL-6R mit dem NH₂-Terminus von humanem IL-6.
- Fig. 2: zeigt die Aminosäure (DNA)-sequenz eines erfindungsgemäßen Fusionspolypeptids H-IL-6. Sequenzen für das Restriktionsenzym Sall (GTCGAC), das Signalpeptid (MLAVGCALLAALLAAPGAA) und den Linker (RGGGGSGGGGSVE) sind angegeben. Der Linker verbindet den COOH-Terminus von humanem slL-6R mit dem NH₂-Terminus von humanem IL-6.
- Fig. 3: zeigt die Aminosäuresequenz des in einem erfindungsgemäßen Fusionspolypeptid H-IL-6 vorliegenden IL-6-Polypeptids.
- Fig. 4: zeigt die Expansions- und Koloniebildungsfähigkeit eines erfindungsgemäßen Fusionspolypeptids H-IL-6.

Die Erfindung wird durch die nachfolgenden Beispiele erläutert.

### Beispiel 1: Herstellung einer erfindungsgemäßen DNA

Es wurde die DNA von Fig. 1 hergestellt. Dazu wurde humane IL-6R cDNA (Schooltink et al., Biochem. J. (1991) 277, 659-664) verwendet. Diese cDNA wurde in das Expressionsplasmid pCDM8 über die Restriktionsstelle Xho I einkloniert (Müllberg et al., Eur. J. Immunol. (1993) 23, 473-480). Mit Hilfe einer Polymerasekettenreaktion (PCR) wurde unter Verwendung der Primer (1) (pCDM8 5' Primer: 5' TAATACGACTCACTATAGGG3') und Primer (2) (sIL-6R 3' Primer: 5'CCGCTCGAGCTGGAGGACTCCTGGA 3') bei Standardbedingungen ein slL-6R Fragment generiert, das nach Schneiden mit den Restriktionsenzymen Hind III und Xho I in das geöffnete Plasmid pCDM8 einkloniert wurde. Es entstand das Plasmid pCDM8-sIL-6R. Anschließend wurde eine zweite PCR Reaktion mit IL-6 cDNA, die ebenfalls in das Expressionsplasmid pCDM8 unter Verwendung von Xho I einkloniert worden war, durchgeführt. Es wurden die Primer (3) (IL-6-5' Primer: 5' CGGCTCGAGCCAGTACCCCCAGGAGAA3') und Primer (4) (pCDM8 3' Primer: 5'CCACAGAAGTAAGGTTCCTT3') verwendet. Das PCR Produkt wurde mit den Restriktionsenzymen Xho I und Not I geschnitten und in das Plasmid pCDM8-sIL-6R einkloniert. Es entstand das Plasmid pCDM8-sIL-6R-IL-6. Anschließend wurde ein synthetischer Linker hergestellt, der aus zwei Oligonukleotiden bestand: Primer (5) (5'TCGAG-GAGGTGGAGGTTCTGGAGGTGGAGGTTCTGGAGGTGGAGGTTCTG3') und Primer (6) (5'TCGACAGAACCTCCACCTCCAGAACCTCCACCTCCA-GAACCTCCACCTCC3'. Die Oligonukleotide (5) und (6) wurden nach Standardmethoden zu einem Doppelstrang zusammengefügt und anschließend in das mit dem Restriktionsenzym Xho I verdaute Plasmid pCDM8-sIL-6R-IL-6 einkloniert. Es entstand das Plasmid pCDM8-H-IL-6.

### Beispiel 2: Herstellung einer erfindungsgemäßen DNA

Es wurde die DNA von Fig. 2 hergestellt. Hierzu wurde vorgegangen, wie in Beispiel 1 beschrieben. Als Primer (5) und (6) wurden jedoch verwendet: Primer (5) (5'TCGAGGAGGTGGAGGTTCTGGAGGTGGAGGTTCTG3') und Primer (6) (5'TCGACAGAACCTCCACCTCCAGAACCTCCACCTCC3'. Es wurde das Plasmid pCDM8-H-IL-6-(2) erhalten.

### Beispiel 3: Expression eines erfindungsgemäßen Fusionspolypeptids

COS-7-Zellen wurden mit pCDM8-H-IL-6 von Beispiel 1 bzw. pCDM8-H-IL-6(2) von Beispiel 2 mit Hilfe der Elektroporation transfiziert. Es wurden 10⁷ COS-7 Zellen mit 20µg Plasmid mit Hilfe eines Gene-Pulsers (Bio-Rad) bei 960µF und 230 V elektroporiert. 48 h nach der Transfektion wurden die Zellen metabolisch mit [³⁵S]-Cystein/Methionin 4 h radioaktiv markiert und 2 h mit nicht radioaktiv markierten Aminosäuren inkubiert. Der Überstand aus Zellysat und Zellüberstand wurde nach Standardmethoden (Müllberg et al., Eur. J. Immunol. (1993) 23, 473-480) mit einem anti-IL-6 Antikörper immunpräzipitiert und nach SDS-Gelelektrophorese durch Autoradiographie sichtbar gemacht. Transfizierte COS-7 Zellen sezernierten ein 70-75 kDa Protein, das von einem anti-IL-6 Antikörper erkannt und nicht von untransfizierten Zellen gebildet wurde.

Überstände von transfizierten COS-7 Zellen wurden durch SDS-Gelelektrophorese aufgetrennt, auf Nitrozellulose übertragen und mit einen anti-IL-6 Antikörper detektiert. Wiederum exprimierten transfizierte COS-7 Zellen ein 70-75 kDa Protein, das von einem anti-lL-6 Antikörper erkannt wurde.

Überstände von transfizierten COS-7 Zellen wurden mit Hilfe eines kommerziellen ELISA auf IL-6 (CLB, Amsterdam) und sIL-6R (Seromed, Gießen) untersucht. Mit beiden ELISAs wurde H-IL-6 detektiert. Die Konzentration von H-IL-6 im Zellüberstand betrug etwa 1 µg/ml.

### Beispiel 4: Stimulation der Haptoglobin-Expression durch ein erfindungsgemässes Fusionspolypeptid

Es wurden die humanen Hepatomazellinien HepG2, HepG2-IL-6 und HepG2-PDI verwendet.

HepG2 Zellen (ATCC HB 8065) werden durch IL-6, nicht aber durch slL-6R stimuliert, Haptoglobin zu exprimieren.

HepG2-IL-6 Zellen wurden durch stabile Transfektion von HepG2 Zellen mit einem humanen IL-6-Expressionsplasmid erhalten. Diese Zellen regulieren aufgrund der IL-6 Expression endogenes IL-6R herunter und exprimieren somit kein IL-6R. HepG2-IL-6 Zellen werden nicht durch IL-6, aber durch sIL-6R stimuliert, Haptoglobin zu exprimieren.

HepG2-PDI Zellen wurden durch stabile Transfektion von HepG2 Zellen mit einem humanen lL-6-Expressionsplasmid erhalten. Hierzu wies das Expressionsplasmid eine IL-6 cDNA auf, durch die das exprimierte IL-6 Protein ein COOHterminales Retentionssignal für das Endoplasmatische Retikulum (ER) enthielt. Daraus resultierte, daß diese Zellen nicht durch das exprimierte IL-6, sondern auch IL-6R im ER zurückhielten. Im Gegensatz zu HepG2-IL-6 Zellen sezernieren aber HepG2-PDI Zellen kein IL-6 und können nur durch die Kombination von IL-6 und slL-6R stimuliert werden, Haptoglobin zu exprimieren.

Die vorstehenden Hepatomazellinien wurden nach Standardbedingungen in 96er Zellkulturplatten kultiviert (Rose-John et al., J. Biol. Chem. 268 (1993), 22084-22091). Die Zellen wurden mit IL-6, slL-6R, IL-6 + slL-6R bzw. Zellüberständen aus mit pCDM8-H-IL-6, pCDM8-H-IL-6(2) bzw. pCDM8 transfizierten COS-7 Zellen von Beispiel 3 18 h stimuliert. Der Zellüberstand wurde geerntet und die Haptoglobinkonzentration im Überstand mittels ELISA bestimmt (vgl. Tabelle I).

**Tabelle I**

| **Stimulation der Haptoglobin-Expression** | | | | | |
|---|---|---|---|---|---|
| | **IL-6** | **sIL-6R** | **IL-6 + sIL-6R** | **H-IL-6** | **Kontrolle** |
| HepG2 | + | - | + + | + + + | - |
| HepG2-IL-6 | - | + + | + + | + + + | - |
| HepG2-PDI | - | - | + + | + + + | - |

Es zeigte sich, daß ein erfindungsgemäßes Fusionspolypeptid, H-IL-6, in der Lage ist, die Expression von Haptoglobin in Zellen zu stimulieren, d.h. die Wechselwirkungen zwischen Proteinen zu beeinflussen.

### Beispiel 5: Expansion und Koloniebildung von humanen CD34⁺-Zellen durch ein erfindungsgemäßes Fusionspolypeptid

Aus humanem Knochenmark bzw. aus Blut von Patienten, deren Stammzellen durch Injektion von G-CSF mobilisiert worden waren, wurden Zellen isoliert, die den Oberflächenmarker CD34 exprimieren. 6000 dieser Zellen wurden in 3ml Medium in Zellkulturgefäße platiert. Nach zwei Wochen zeigte es sich, daß eine Inkubation der Zellen mit den Zytokinen SCF, IL-3 und H-IL-6 (erfindungsgemäßes Fusionspolypeptid), wie auch mit SCF, IL-3 und IL-6 eine starke Proliferation verursachte. Von den entstandenen Zellen wurden 1000 Zellen in neue Zellkulturgefäße platiert. Nach zwei Wochen in einem standartisierten Kolonielnduktions-Versuch waren die mit SCF, IL-3 und H-IL-6 behandelten Zellen in der Lage, etwa dreimal mehr Kolonien zu bilden als mit SCF, IL-3 und IL-6 behandelte Zellen.

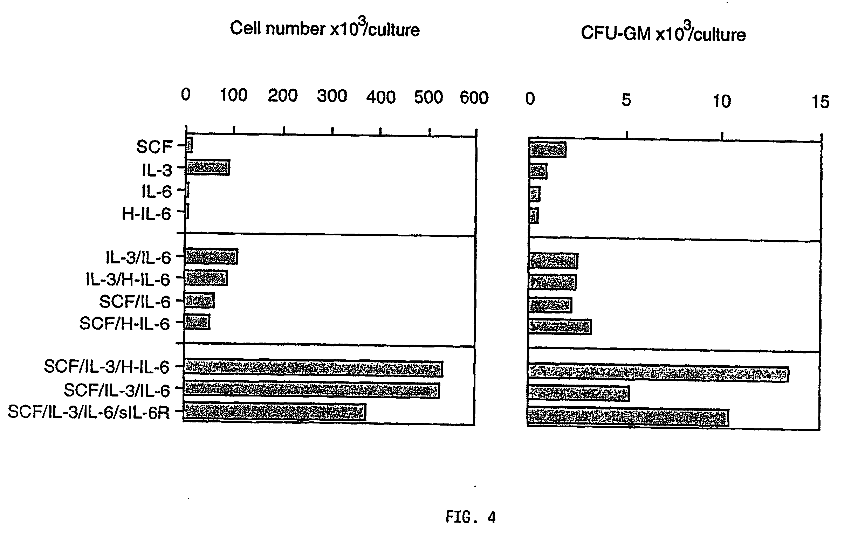

Dieses Ergebnis zeigt, daß Zellen, die durch ein erfindungsgemäßes Fusionspolypeptid H-IL-6 stimuliert wurden, ein höheres koloniebildendes Potential besitzen als durch IL-6 stimulierte Zellen (vgl. Fig. 4).

## Patentansprüche

1. Fusionspolypeptid, umfassend zwei, eine Affinität zueinander aufweisende, Polypeptide, wobei das eine Polypeptid ein Zytokin der Interleukin-6-Familie und das andere der dazugehörige Rezeptor ist und die Polypeptide über einen Polypeptid-Linker miteinander verbunden sind.

2. Fusionspolypeptid nach Anspruch 1, **dadurch gekennzeichnet, daß** der Rezeptor in Form seiner den Liganden bindenden Untereinheit vorliegt.

3. Fusionspolypeptid nach Anspruch 1 oder 2, **dadurch gekennzeichnet, daß** der Ligand in Form seiner den Rezeptor bindenden Untereinheit vorliegt.

4. Fusionspolypeptid nach einem der Ansprüche 1-3, **dadurch gekennzeichnet, daß** der Zytokin-Rezeptor ein IL-6 Rezeptor und das Zytokin ein IL-6 ist.

5. Fusionspolypeptid nach einem der Ansprüche 1-3, **dadurch gekennzeichnet, daß** der Zytokin-Rezeptor ein CNTF-Rezeptor und das Zytokin ein CNTF ist.

6. Fusionspolypeptid nach einem der Ansprüche 1-3, **dadurch gekennzeichnet, daß** das Zytokin ein IL-11 ist.

7. DNA, kodierend für das Fusionspolypeptid nach einem der Ansprüche 1-6.

8. Expressionsplasmid, umfassend die DNA nach Anspruch 7.

9. Transformante, enthaltend das Expressionsplasmid nach Anspruch 8.

10. Arzneimittel, enthaltend das Fusionspolypeptid nach einem der Ansprüche 1-6 oder die DNA nach Anspruch 7.

## Claims

1. A fusion polypeptide, comprising two polypeptides having affinity with respect to each other, wherein one polypeptide is a cytokine of the interleukin-6 family and the other is the associated receptor and the polypeptides are linked with each other via a polypeptide linker.

2. The fusion polypeptide according to claim 1, **characterized in that** the receptor is available as its subunit binding the ligand.

3. The fusion polypeptide according to claim 1 or 2, **characterized in that** the ligand is available as its subunit binding the receptor.

4. The fusion polypeptide according to any of claims 1 to 3, **characterized in that** the cytokine receptor is an IL-6 receptor and the cytokine is IL-6.

5. The fusion polypeptide according to any of claims 1 to 3, **characterized in that** the cytokine receptor is a CNTF receptor and the cytokine is CNTF.

6. The fusion polypeptide according to any of claims 1 to 3, **characterized in that** the cytokine is IL-11.

7. DNA coding for the fusion polypeptide according to any of claims 1 to 6.

8. Expression plasmid comprising the DNA according to claim 7.

9. Transformant containing the expression plasmid according to claim 8.

10. A medicament containing the fusion polypeptide according to any of claims 1 to 6 or the DNA according to claim 7.

## Revendications

1. Polypeptide de fusion comprenant deux polypeptides doués d'affinité l'un pour l'autre, dont l'un est une cytokine de la famille de l'interleukine-6 et l'autre est le récepteur correspondant, ces polypeptides étant liés l'un à l'autre par l'intermédiaire d'un lieur de polypeptides.

2. Polypeptide de fusion suivant la revendication 1, **caractérisé en ce que** le récepteur est présent sous forme de sa sous-unité fixant le ligand.

3. Polypeptide de fusion suivant la revendication 1 ou 2, **caractérisé en ce que** le ligand est présent sous forme de sa sous-unité fixant le récepteur.

4. Polypeptide de fusion suivant l'une des revendications 1 à 3, **caractérisé en ce que** le récepteur de cytokine est le récepteur IL-6 et la cytokine est l'interleukine IL-6.

5. Polypeptide de fusion suivant l'une des revendications 1 à 3, **caractérisé en ce que** le récepteur de cytokine est un récepteur de CNTF et la cytokine est un facteur CNTF.

6. Polypeptide de fusion suivant l'une des revendications 1 à 3, **caractérisé en ce que** la cytokine est une cytokine IL-11.

7. ADN codant le polypeptide de fusion suivant l'une des revendications 1 à 6.

8. Plasmide d'expression comprenant l'ADN suivant la revendication 7.

9. Transformants contenant le plasmide d'expression suivant la revendication 8.

10. Médicament contenant le polypeptide de fusion suivant l'une des revendications 1 à 6 ou l'ADN suivant la revendication 7.
